(19) Europäisches Patentamt
European Patent Office
Office européen des brevets

(11) Publication number: **0 622 370 A2**

# (12) EUROPEAN PATENT APPLICATION

(21) Application number: **94111438.1**

(22) Date of filing: **21.07.88**

(51) Int. Cl.5: **C07D 493/08**, C07D 495/08, C07D 497/08, A01N 43/90, C07D 305/06, C07D 305/08, C07C 323/12, //(C07D493/08, 319:00,319:00),(C07D495/08, 339:00,339:00)

(30) Priority: **22.07.87 GB 8717274**
**07.10.87 GB 8723488**
**26.04.88 GB 8809851**

(43) Date of publication of application:
**02.11.94 Bulletin 94/44**

(60) Publication number of the earlier application in accordance with Art.76 EPC: **0 300 797**

(84) Designated Contracting States:
**AT BE CH DE ES FR GB GR IT LI LU NL SE**

(71) Applicant: **ROUSSEL-UCLAF**
**35, Boulevard des Invalides**
**F-75007 Paris (FR)**

(72) Inventor: **Weston, John Bernard**
**ROUSSEL-UCLAF,**
**102, route de Noisy**
**F-93235 Romainville Cédex (FR)**
Inventor: **Larkin, John Patrick**
**ROUSSEL-UCLAF,**
**102, route de Noisy**
**F-93235 Romainville Cédex (FR)**
Inventor: **Smith, Ian Harold**
**The Wellcome Research Laboratories,**
**Langley Court**
**Beckenham, Kent BR3 3BS (GB)**

(74) Representative: **Vieillefosse, Jean-Claude et al**
**ROUSSEL UCLAF,**
**111 Route de Noisy**
**F-93235 Romainville Cédex (FR)**

## (54) Pesticidal compounds.

(57) New compounds of the formula (I):

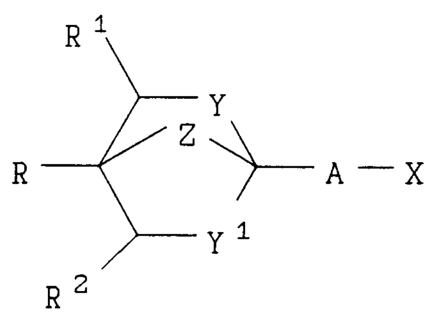

(I)

wherein R is a $C_{2-10}$ non-aromatic hydrocarbyl group optionally substituted by, or a methyl group substituted by cyano, halo, $C_{1-4}$ alkoxy optionally substituted by halo, or a group $S(O)_mR^3$, or R is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mR^3$ ;

$R^1$ and $R^2$ may be the same or different, and each is hydrogen, halo, or a $C_{1-3}$ aliphatic group optionally



Rank Xerox (UK) Business Services
(3.10/3.09/3.3.4)

substituted by halo, cyano, $C_{1-5}$ carbalkoxy, $C_{1-4}$ alkoxy, or a group $S(O)_{m'}R^4$, or $R^1$ and R and the carbon atoms to which they are attached form a $C_{5-7}$ carbocyclic ring optionally substituted by halo, or a $C_{1-3}$ aliphatic or alkoxy group;

A is a $C_{5-8}$ non-aromatic hydrocarbyl group which contains one hetero atom selected from oxygen or sulphur and terminates in a C≡C fragment adjacent to X;

X is hydrogen;

Y and Y1 are the same or different and are each selected from oxygen and $S(O)_{n'}$ and Z is $CH_2O$ or $CH_2S(O)_{n''}$;

the methods for their preparation, the compositions containing them and their use in the control of pests.

The present invention relates to novel chemical compounds having pesticidal activity, to methods for their preparation, to compositions containing them and to their use in the control of pests. More particularly the invention relates to a class of heterobicycloalkanes.

The use of certain pesticidally active 2,6,7-trioxabicyclo[2.2.2]octanes is disclosed in European Patent Applications Nos. 152229, 211598, 216625, 216624, US Patent 3686224 and J.Agric.Food Chem. 33, 976 (1985). It has now been discovered that derivatives of these compounds have interesting pesticidal activity.

Accordingly, the present invention provides a compound of the formula (I):

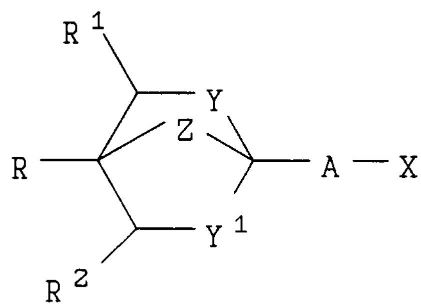

**(I)**

wherein R is a $C_{2-10}$ non-aromatic hydrocarbyl group optionally substituted by, or methyl substituted by cyano, halo, $C_{1-4}$ alkoxy optionally substituted by halo, or a group $S(O)_mR^3$ where $R^3$ is $C_{1-4}$ alkyl optionally substituted by halo and m is 0, 1 or 2, or R is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mR^3$ as defined hereinbefore;

$R^1$ and $R^2$ may be the same or different, and each is hydrogen, halo, or a $C_{1-3}$ aliphatic group optionally substituted by halo, cyano, $C_{1-5}$ carbalkoxy, $C_{1-4}$ alkoxy, or a group $S(O)_{m'}$ $R^4$ wherein m' is O, 1 or 2 and $R^4$ is $C_{1-4}$ alkyl; cyano, gem- dimethyl, or $C_{1-5}$ carbalkoxy, or $R^1$ and R and the carbon atoms to which they are attached form a $C_{5-7}$ carbocyclic ring optionally substituted by halo, or a $C_{1-3}$ aliphatic or alkoxy group;

A is a $C_{5-8}$ non-aromatic hydrocarbyl group which contains one hetero atom selected from oxygen or sulphur and terminates in a C≡C fragment adjacent to X.

X is hydrogen.

Y and $Y^1$ are the same or different and are each selected from oxygen and $S(O)_{n'}$ where n'is 0, 1 or 2; and Z is $CH_2O$ or $CH_2S(O)_{n''}$, wherein n''is 0, 1 or 2; In the definition of Z, the first mentioned atom is adjacent to the 4-position of the bicyclic ring system.

When R is an alkyl, alkenyl or alkynyl, cycloalkyl or cycloalkenyl group this preferably contains up to 6 carbon atoms. Conveniently, when R is substituted there are up to 7 substituents when the substituent is fluoro, 3 substituents when the substituents are chloro or bromo or 1 substituent when this is other than halo.

Suitably, R is an aliphatic or alicyclic group containing between 2 and 8 carbon atoms or phenyl each optionally substituted by cyano, 1 to 7 halo atoms, $C_{1-4}$ alkoxy or a group $S(O)_mR^4$ as hereinbefore defined. Most suitably R is propyl, butyl, pentyl, $C_{2-5}$ alkenyl or alkynyl, cyclopropyl methyl, $C_{3-7}$ cycloalkyl or phenyl each optionally substituted by fluoro, chloro or bromo, for example, n-propyl, n-butyl, i-butyl, sec-butyl, t-butyl, prop-2-enyl, 2-methyl prop-2-enyl, but-3-enyl, phenyl, cyclopentyl or cyclohexyl. Preferably, R is n-propyl, n-butyl, i-butyl, t-butyl or phenyl.

Suitably $R^1$ is hydrogen, halo, cyano, methyl or ethyl each optionally substituted by cyano, methoxy, methylthio, chloro, bromo or fluoro. Most suitably $R^1$ is hydrogen, methyl, cyano, trifluoromethyl or ethyl. Preferably $R^1$ is hydrogen, methyl, cyano or trifluoromethyl.

Suitably $R^2$ is hydrogen, cyano, methyl or trifluoromethyl. Most suitably $R^2$ is hydrogen or methyl. Preferably $R^2$ is hydrogen.

Preferably Z is $CH_2S$ or $CH_2O$.

Suitably Y and $Y^1$ are both selected from oxygen or sulphur.

Preferably A is a $CH_2O(CH_2)_2C{\equiv}C$ group.

The compounds of the formula (I) may exist in a number of isomeric forms. The present invention provides individual isomers of compounds of the formula (I) and mixtures thereof. The present invention also encompasses compounds of the formula (I) containing radioisotopes, particularly those in which one carbon atom is $^{14}C$ or one to three hydrogen atoms are replaced by tritium.

Preferred compounds of the present invention include:
4-t-Butyl-1-[2-(prop-2-ynylthio)ethyl]-2,6,7-trioxa bicyclo[2.2.2]octane,
4-n-Propyl-1-[2-(prop-2-ynylthio)ethyl]-2,6,7-trioxa bicyclo[2.2.2]octane,
4-t-Butyl-1-[2-(prop-2-ynyloxy)ethyl]-2,6,7-trioxa bicyclo[2.2.2]octane,
4-n-Propyl-1-[2-(prop-2-ynyloxy)ethyl]-2,6,7-trioxa bicyclo[2.2.2]octane-3-carbonitrile,
1-(But-3-ynyloxymethyl)-4-n-propyl-2,6,7-trioxa bicyclo[2.2.2]octane,
4-t-Butyl-1-(but-3-ynyloxymethyl)-2,6,7-trioxa bicyclo[2.2.2]octane,
1-(But-3-ynyloxymethyl)-4-n-propyl-2,6,7-trioxa bicyclo[2.2.2]octane-3-carbonitrile,
1-(But-3-ynyloxymethyl)-4-(prop-2-enyl)-2,6,7-trioxa bicyclo[2.2.2]octane-3-carbonitrile,
1-[2-(But-3-ynyloxy)ethyl]-4-n-propyl-2,6,7-trioxa bicyclo[2.2.2]octane,
1-[1-(But-3-ynyloxy)ethyl]-4-n-propyl-2,6,7-trioxa bicyclo[2.2.2]octane,
1-(But-3-ynylthiomethyl)-4-n-propyl-2,6,7-trioxa bicyclo[2.2.2]octane,
4-t-Butyl-1-(but-3-ynylthiomethyl)-2,6,7-trioxa bicyclo[2.2.2]octane,
1-(But-3-ynyloxymethyl)-4-n-propyl-2,6,7-trithia bicyclo[2.2.2]octane.

By the term "hydrocarbyl" group is meant alkyl, alkenyl (including cyclic alkyl and alkenyl, and alkyl and alkenyl substituted by cyclic alkyl and alkenyl), alkynyl, aryl and aralkyl groups. "Hydrocarbyloxy" means a hydrocarbyl group as defined where linked to oxygen. By the term "aliphatic" group is meant an alkyl, alkenyl or alkynyl group. By the term "halo" is meant fluoro, chloro, bromo or iodo.

In a further aspect, the present invention provides a process for the preparation of a compound of the formula (I). The process for the preparation of a compound of the formula (I) may be any method known in the art for preparing analogous compounds, for example:

(i) when Y and $Y^1$ are oxygen and Z is $CH_2O$:

a) by the cyclisation of a compound of the formula (II):

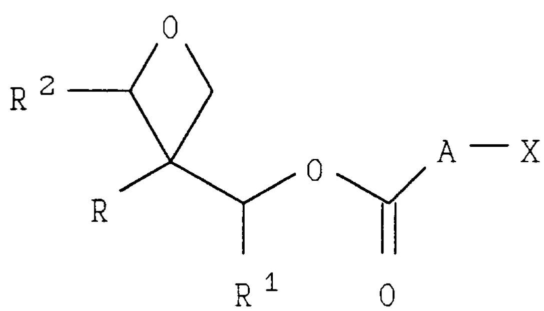

(II)

wherein R to $R^2$, A and X are as hereinbefore defined, in the presence of an acid catalyst. Boron trifluoride etherate is a particularly preferred acid catalyst for this cyclisation which will normally be carried out in an inert solvent, such as a halogenated hydrocarbon, conveniently dichloromethane, at or below ambient temperature, for example between -100 and 50°C and conveniently between -70 and -25°C. The compounds of the formula (II) may be prepared by the reaction of compounds of the formula (III):

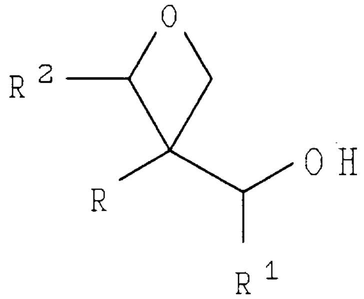

(III)

and (IV):

L-CO-A-X (IV)

wherein R to $R^2$, A and X are as hereinbefore defined and L is a leaving group such as halo or hydroxy. This reaction conveniently takes place under conditions well known to those skilled in the art, for example when L is halo in an inert solvent in the presence of base at a non-extreme temperature and when L is hydroxy in an inert solvent in the presence of a condensing agent at a non extreme temperature. When L is halo, halogenated hydrocarbons, such as dichloromethane, are particularly suitable inert solvents and pyridine is a preferred base; when L is hydroxy, dimethylformamide is a suitable solvent, dicyclohexylcarbodiimide is a preferred condensing agent; and the reaction will conveniently be carried out at between -50 and 100°C, preferably between 0 and 25°C.

The compounds of the formula (III) may be prepared as described in copending European Patent Applications Nos. 211598 and 216624. The compounds of the formula (IV) may be prepared by methods well known to those skilled in the art;

b) when A contains a terminal C≡C fragment, by the reaction of a strong base with a compound of the formula (VII):

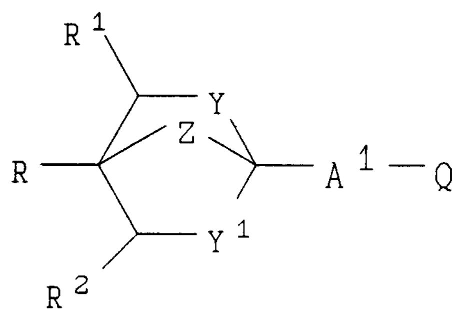

(VII)

wherein R to $R^2$, Y, Y1 and Z are as hereinbefore defined, $A^1$ is defined such that $A^1$-C≡C forms the group A and Q is a group capable of conversion into an ethynyl group, for example a group CH = C-(hal)$_2$, (hal)CH = $CH_2$ or -C(O-PO-(OAlk)$_2$) = $CH_2$, wherein hal is chloro or bromo, Alk is $C_{1-4}$ alkyl. The reaction is conveniently carried out by methods well known to those skilled in the art, for example when Q is a group -CH = C(hal)$_2$, at about or below room temperature, for example between -70°C and 25°C, in an inert solvent, conveniently an ether such as tetrahydrofuran. Butyl lithium is a convenient strong base for use in this reaction. The starting material of the formula (VII) may be prepared by the reaction of a compound of the formula (VIII):

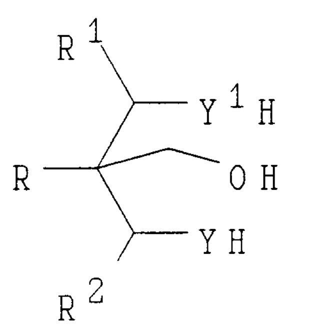

(VIII)

(ii) when n' is O, $Y^1$ = O or S, Y = O or S and Z = $CH_2S$ or $CH_2O$, by the reaction of a compound of the formula (XIV)

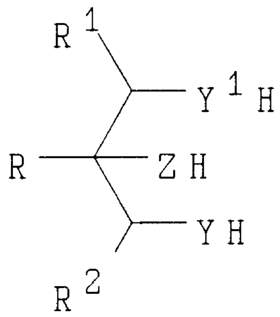

(XIV)

with a compound of the formula $(Alko)_3C$-A-X, wherein R, $R^1$, $R^2$, A, X, Y, $Y^1$ and Z are as hereinbefore defined and Alk is a $C_{1-4}$ alkyl group. The condensation takes place in the presence of an acid catalyst for example a mineral acid such as concentrated hydrochloric acid or boron trifluoride etherate and/or p-toluenesulphonic. The reaction is conveniently carried out without a solvent, but an inert solvent, conveniently a chlorinated hydrocarbon such as dichloromethane may be added. The reaction can also be carried out in methanol containing hydrogen chloride. The reaction is conveniently carried out at a non-extreme temperature, for example between -70°C and 150°C and normally between -10°C and 150°C. The compounds of the formula (XIV) may be prepared as described in European Patent Application No 216624. Compounds of the formula (XIV) wherein Y = $Y^1$ = S, Z = $CH_2S$ and $R^1$ = $R^2$ = H may also be prepared by the method described by G. R. Franzen and G. Binsch, J. Amer. Chem. Soc., 1973, 95, 175 and D.J. Martin and C.R. Creco, J. Org. Chem., 1968, 33, 1275. The compounds of the formula $(AlkO)_3C$-A-X may be prepared by a general procedure for the synthesis of orthoesters and is described by S.M. McElvain and R.E.Stam, J. Amer. Chem. Soc., 1955, 77, 4571;

(iii) when Z = $CH_2S$ or $CH_2O$ and Y and $Y^1$ are sulphur, by reaction of a compound of the formula (XV):

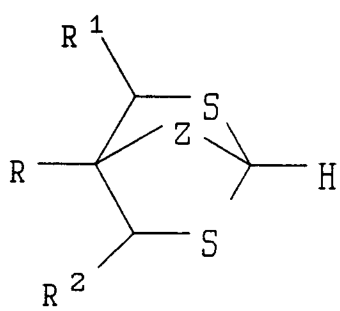

(XV)

with a compound $L^2$-A-X wherein R to $R^2$, A and X are as herein before defined, $Z^1$ is $CH_2S$ or $CH_2O$ and $L^2$ is a leaving group eg.halo. The reaction is suitably carried out in the presence of a strong base, such as butyllithium, in an inert solvent, such as an ether and conveniently tetrahydrofuran at a non-extreme temperature, such as between -70° and 30°C. The compound of the formula (XV) can be prepared by the reaction of an analogous compound of the formula (XIV) with $HC(OAlk)_3$ under the conditions described for reaction (ii) above.

(iv) By the interconversion of compounds of the formula (I); for example:

(a) when it is required to prepare a compound of the formula (I) wherein A is saturated or contains a double bond, by the reduction of the corresponding double or triple bond respectively. This reaction conveniently takes place by hydrogenation in the presence of a catalyst, for example palladium on charcoal, or when a triple bond is being reduced and it is required to stop the reduction once a double bond is formed and not proceed to the fully saturated compound, in the presence of such a catalyst poisoned by, for example, barium sulfate. The reaction is conventiently carried out in a suitable solvent for hydrogenation experiments such as methanol or ethyl acetate. the reaction is usually carried out at a non-extreme temperature, for example between 5° and 50°C and normally at 25°C.

(b) when A contains a terminal C≡C fragment and X is hydrogen, by desilylation of a compound of the formula (XVII):

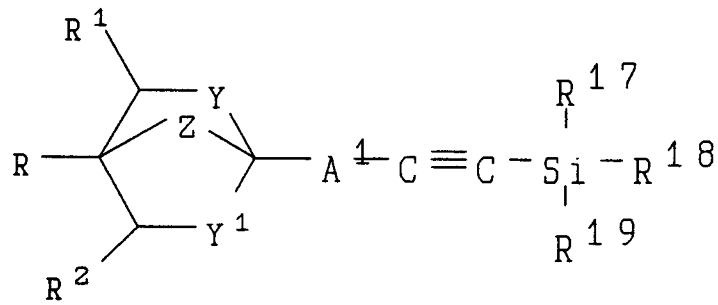

(XVII)

wherein R, $R^1$, $R^2$, $R^{17}$, $R^{18}$, $R^{19}$, Y, $Y^1$, Z and $A^1$ are as hereinbefore defined. This reaction may be carried out by methods well known to those skilled in the art, for example by reaction with tetrabutylammonium fluoride in an ether, such as tetrahydrofuran, at a non-extreme temperature, for example between 0° and 70°C and conveniently at 25°C.

Novel chemical intermediates also form an important aspect of the present invention. Preferred intermediates include those of the formulas (II), (VII), (XIV) and (XV).

The compounds of formula (I) may be used to control pests such as arthropods e.g. insect and acarine pests, and helminths, i.e. nematodes. Thus, the present invention provides a method for the control of arthropods and/or helminths which comprises administering to the arthropod and/or helminth or to their environment an arthropodically effective amount of a compound of the formula (I). The present invention also provides a method for the control and/or eradication of arthropod and/or helminth infestations of animals (including humans) and/or of plants, (including trees) and/or stored products which comprises administering to the animal or locus an effective amount of a compound of the formula (I). The present invention further provides for the compounds of the formula (I) for use in human and veterinary medicine, in public health control and in agriculture for the control of arthropod and/or helminth pests.

The compounds of formula (I) are of particular value in the protection of field, forage, plantation, glasshouse, orchard and vineyard crops, of ornamentals and of plantation and forest trees, for example, cereals (such as maize, wheat, rice, sorghum), cotton, tobacco, vegetables and salads (such as beans, cole crops, curcurbits, lettuce, onions, tomatoes and peppers), field crops (such as potato, sugar beet, ground nuts, soyabean, oil seed rape), sugar cane, grassland and forage (such as maize, sorghum, lucerne), plantations (such as of tea, coffee, cocoa, banana, oil palm, coconut, rubber, spices), orchards and groves (such as of stone and pip fruit, citrus, kiwifruit, avocado, mango, olives and walnuts), vineyards, ornamental plants, flowers and shrubs under glass and in gardens and parks, forest trees (both deciduous and evergreen) in forests, plantations and nurseries. They are also valuable in the protection of timber (standing, felled, converted, stored or structural) from attack by sawflies (e.g. Urocerus) or beetles (e.g. scolytids, platypodids, lyctids, bostrychids, cerambycids, anobiids). They have applications in the protection of stored products such as grains, fruits, nuts, spices and tobacco, whether whole, milled or compounded into products, from moth, beetle and mite attack. Also protected are stored animal products such as skins, hair, wool and feathers in natural or converted form (e.g. as carpets or textiles) from moth and beetle attack; also stored meat and fish from beetle, mite and fly attack. The compounds of general formula (I) are of particular value in the control of arthropods or helminths which are injurious to, or spread or act as vectors of diseases in man and domestic animals, for example those hereinbefore mentioned, and more especially in the control of ticks, mites, lice, fleas, midges and biting, nuisance and myiasis flies. The compounds of Formula (I) may be used for such purposes by application of the compounds themselves or in diluted form in known fashion as a dip, spray, fog, lacquer, foam, dust, powder, aqueous suspension, paste, gel, cream, shampoo, grease, combustible solid, vapourising mat, combustible coil, bait, dietary supplement, wettable powder, granule, aerosol, emulsifiable concentrate, oil suspensions, oil solutions, pressure-pack, impregnated article, pour on formulation or other standard formulations well known to those skilled in the art. Dip concentrates are not applied per se, but diluted with water and the animals immersed in a dipping bath containing the dip wash. Sprays may be applied by hand or by means of a spray race or arch. The animal, soil, plant or surface being treated may be saturated with the spray by means of high volume application or superficially coated with the spray by means of light or ultra low volume application. Aqueous suspensions may be applied in the same manner as sprays or dips. Dusts may be distributed by means of a powder applicator or, in the case of animals, incorporated in perforated bags attached to trees or rubbing bars. Pastes, shampoos and greases may be applied manually or distributed over the surface of an inert material, such as that against which animals rub and transfer the material to their skins. Pour-on formulations are

dispensed as a unit of liquid of small volume on to the backs of animals such that all or most of the liquid is retained on the animals.

The compounds of Formula (I) may be prepared either as formulations ready for use on the animals, plants or surface or as formulations requiring dilution prior to application, but both types of formulation comprise a compound of Formula (I) in intimate admixture with one or more carriers or diluents. The carriers may be liquid, solid or gaseous or comprise mixtures of such substances, and the compound of Formula (I) may be present in a concentration of from 0.025 to 99% w/v depending upon whether the formulation requires further dilution. Dusts, powders and granules and other solid formulations comprise the compound of Formula (I)in intimate admixture with a powdered solid inert carrier for example suitable clays, kaolin, bentonite, attapulgite, adsorbent carbon black, talc, mica, chalk, gypsum, tricalcium phosphate, powdered cork, magnesium siliate, vegetable carriers, starch and diatomaceous earths. Such solid formulations are generally prepared by impregnating the solid diluents with solutions of the compound of formula (I) in volatile solvents, evaporating the solvents and, if desired grinding the products so as to obtain powders and, if desired, granulating, compacting or encapsulating the products. Sprays of a compound of Formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and other solvents known in the formulating art. The concentration of emulsifiers may be varied within wide limits but is preferably in the range of 5 to 25% w/v and the emulsifiers are conveniently non-ionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates. Cationic emulsifiers include benzalkonium chloride and quaternary ammonium ethosuphates. Amphoteric emulsifiers include carboxymethylated oleic imidazoline and alkyl dimethyl betain. Vaporising mats normally comprise cotton and cellulose mix compressed into a board of approximately 35 x 22 x 3mm dimensions, treated with up to 0.3ml of concentrate comprising the active ingredient in an organic solvent and optionally an antioxidant, dye and perfume. The insecticide is vaporised using a heat source such as an electrically operated mat heater. Combustible solids normally comprise of wood powder and binder mixed with the active ingredient and formed into shaped (usually coiled) strips. Dye and fungicide may also be added. Wettable powders comprise an inert solid carrier, one or more surface active agents, and optionally stabilisers and/or anti-oxidants. Emulsifiable concentrates comprise emulsifying agents, and often an organic solvent, such as kerosene, ketones, alcohols, xylenes, aromatic naphtha, and other solvents known in the art. Wettable powders and emulsifiable concentrates will normally contain from 5 to 95% by weight of the active ingredient, and are diluted, for example with water, before use. Lacquers comprise a solution of the active ingredient in an organic solvent, together with a resin, and optionally a plasticiser. Dip washes may be prepared not only from emulsifiable concentrates but also from wettable powders, soap based dips and aqueous suspensions comprising a compound of Formula (I) in intimate admixture with a dispersing agent and one or more surface active agents. Aqueous suspensions of a compound of Formula (I) may comprise a suspension in water together with suspending, stabilizing or other agents. The suspensions or solutions may be applied per se or in a diluted form in known fashion. Greases (or ointments) may be prepared from vegetable oils, synthetic esters of fatty acids or wool fat together with an inert base such as soft paraffin. A compound of Formula (I) is preferably distributed uniformly through the mixture in solution or suspension. Greases may also be made from emulsifiable concentrates by diluting them with an ointment base. Pastes and shampoos are also semi-solid preparations in which a compound of Formula (I) may be present as an uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of Formula (I) required for treatment. Aerosol sprays may be prepared as a simple solution of the active ingredient in the aerosol propellant and co-solvent such as halogenated alkanes and the solvents referred to above, respectively. Pour-on formulations may be made as a solution or suspension of a compound of Formula (I) in a liquid medium. An avian or mammal host may also be protected against infestation of acarine ectoparasites by means of carrying a suitably-moulded, shaped plastics article impregnated with a compound of Formula (I). Such articles include impregnated collars, tags, bands, sheets and strips suitably attached to appropriate parts of the body. Suitably the plastics material is a polyvinyl chloride (PVC). The concentration of the compound of Formula (I) to be applied to an animal, premises or outdoor areas will vary according to the compound chosen, the interval between treatments, the nature of the formulation and

the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% w/w except for undiluted formulations such as pour-on formulations which in general will be deposited at a concentration in the range from 0.1 to 20.0% and preferably 0.1 to 10%. The amount of compound to be applied to stored products in general will lie in the range of from 0.1 to 20ppm. Space sprays may be applied to give an average initial concentration of 0.001 to 1mg of compound of formula (I) per cubic metre of treated space. The compounds of formula (I) are also of use in the protection and treatment of plant species, in which case an effective insecticidal, acaricidal or nematocidal amount of the active ingredient is applied. The application rate will vary according to the compound chosen, the nature of the formulation, the mode of application, the plant species, the planting density and likely infestation and other like factors but in general, a suitable use rate for agricultural crops is in the range 0.001 to 3kg/Ha and preferably between 0.01 and 1kg/Ha. Typical formulations for agricultural use contain between 0.0001% and 50% of a compound of formula (I) and conveniently between 0.1 and 15% by weight of a compound of the formula (I). Dusts, greases, pastes and aerosol formulations are usually applied in a random fashion as described above and concentrations of 0.001 to 20% w/v of a compound of Formula (I) in the applied formulation may be used. The compounds of formula (I) have been found to have activity against the common housefly (Musca domestica). In addition, certain compounds of formula (I) have activity against other arthropod pests including Myzus persicae, Tetranychus urticae, Plutella xylostella, Culex spp. Tribolium castaneum, Sitophilus granarius, Periplaneta americana and Blattella germanica. The compounds of formula (I) are thus useful in the control of arthropods e.g. insects and acarines in any environment where these constitute pests, e.g. in agriculture, in animal husbandry, in public health control and in domestic situations. Insect pests include members of the orders Coleoptera (e.g. Anobium, Ceutorhynchus, Rhynchophorus, Cosmopolites, Lissorhoptrus, Meligethes, Hypothenemus, Hylesinus, Acalymma, Lema, Psylliodes, Leptinotarsa, Gonocephalum, Agriotes, Dermolepida, Heteronychus, Phaedon, Tribolium, Sitophilus, Diabrotica, Anthonomus or Anthrenus spp.), Lepidoptera (e.g. Ephestia, Mamestra, Earias, Pectinophora, Ostrinia, Trichoplusia, Pieris, Laphygma, Agrotis, Amathes, Wiseana, Tryporysa, Diatraea, Sporganothis, Cydia, Archips, Plutella, Chilo, Heliothis, Spodoptera or Tineola spp.), Diptera (e.g. Musca, Aedes, Anopheles, Culex, Glossina, Simulium, Stomoxys, Haematobia, Tabanus, Hydrotaea, Lucilia, Chrysomia, Callitroga, Dermatobia, Gasterophilus, Hypoderma, Hylemyia, Atherigona, Chlorops, Phytomyza, Ceratitis, Liriomyza and Melophagus spp.), Phthiraptera (Malophaga e.g. Damalina spp., Anoplura e.g. Linognathus and Haematopinus spp.), Hemiptera (e.g. Aphis, Bemisia, Phorodon, Aeneolamia, Empoasca, Parkinsiella, Pyrilla, Aonidiella, Coccus, Pseudococus, Helopeltis, Lygus, Dysdercus, Oxycarenus, Nezara, Aleurodes, Triatoma, Psylla, Mysus, Megoura, Phylloxera, Adelyes, Niloparvata, Nephrotetix or Cimex spp.), Orthoptera (e.g. Locusta, Gryllus, Acheta or Schistocerca spp.), Dictyoptera (e.g. Blattella, Periplaneta or Blatta spp.), Hymenoptera (e.g. Athalia, Cephus, Atta, Solenopsis or Monomorium spp.), Isoptera (e.g. Odontotermes and Reticulitermes spp.), Siphonaptera (e.g. Ctenocephalides or Pulex spp.), Thysanura (e.g. Lepisma spp.), Dermaptera (e.g. Forficula spp.), Pscoptera (e.g. Peripsocus spp.) and Thysanoptera (e.g. Thrips tabaci). Acarine pests include ticks, e.g. members of the genera Boophilus, Ornithodorus, Rhipicephalus, Amblyomma, Hyalomma, Ixodes, Haemaphysalis, Dermacentor and Anocentor, and mites and manges such as Acarus, Tetranychus, Psoroptes, Notoednes, Sarcoptes, Psorergates, Chorioptes, Eutrombicula, Demodex, Panonychus, Bryobia, Eriophyes, Blaniulus, Polyphagotarsonemus, Scutigerella, and Oniscus spp. Nematodes which attack plants and trees of importance to agriculture, forestry, horticulture either directly or by spreading bacterial, viral, mycoplasma or, fungal diseases of the plants, include root-knot nematodes such as Meloidogyne spp. (e.g. M. incognita); cyst nematodes such as Globodera spp. (e.g. G. rostochiensis); Heterodera spp. (e.g. H. avenae); Radopholus spp. (e.g. R. similis); lesion nematodes such as Pratylenchus spp. (e.g. P. pratensis); Belonolaimus spp. (e.g. B. gracilis); Tylenchulus spp. (e.g. T. semipenetrans); Rotylenchulus spp. (e.g. R. reniformis); Rotylenchus spp. (e.g. R. robustus); Helicotylenchus spp. (e.g. H. multicinctus); Hemicycliophora spp. (e.g. H. gracilis); Criconemoides spp. (e.g. C. similis); Trichodorus spp. (e.g. T. primitivus); dagger nematodes such as Xiphinema spp. (e.g. X. diversicaudatum), Longidorus spp (e.g. L. elongatus); Hoplolaimus spp. (e.g. H. coronatus); Aphelenchoides spp. (e.g. A. ritzema-bosi, A. besseyi); stem and bulb eelworms such as Ditylenchus spp. (e.g. D. dipsaci).

Compounds of the invention may be combined with one or more other pesticidally active ingredients (for example pyrethroids, carbamates and organophosphates) and/or with attractants, repellents, bacteriocides, fungicides, nematocides, anthelmintics and the like. Furthermore, it has been found that the activity of the compounds of the invention may be enhanced by the addition of a synergist or potentiator,

for example: one of the oxidase inhibitor class of synergists, such as piperonyl butoxide or propyl 2-propynylphenylphosphonate; a second compound of the invention; or a pyrethroid pesticidal compound. When an oxidase inhibitor synergist is present in a formula of the invention, the ratio of synergist to compound of Formula (I) will be in the range 25:1-1:25 e.g. about 10:1. Stabilisers for preventing any chemical degradation which may occur with the compounds of the invention include, for example, antioxidants (such as tocopherols, butylhydroxyanisole and butylhydroxytoluene) and scavengers (such as epichlorhydrin) and organic or inorganic bases e.g. trialkylamines such as triethylamine which can act as basic stabilisers and as scavengers.

The following Examples illustrate preferred aspects of the invention. All temperatures are in degrees Celsius.

**Example I : 4-n-Propyl-1-[2-(prop-2-ynylthio)ethyl]-2,6,7-trioxa bicyclo[2.2.2]octane**

(i) To a stirred mixture of n-valeraldehyde (172 g) and water (2 l) was added solid calcium hydroxide (112 g) and formaldehyde solution (1.4 l of 40% aqueous solution). The reaction temperature was maintained below 40°C and the addition took about 45 minutes. The mixture was then maintained at 60°C for 5 hours. The reaction mixture was filtered through Kieselguhr and the filtrates were evaporated in vacuo.

The residue was treated with hot methanol (2 l) and the mixture was filtered through Kieselguhr. The filtrates were evaporated in vacuo. A viscous oily product was obtained (458 g) and was purified as follows:

A solution of the crude product and acetic acid (200 ml) was stirred at room temperature. Acetic anhydride (1.2 l) was added over 4 hours. The temperature rose to 65°C. Stirring was continued for 12 hours. The reaction mixture was added over 3 hours to cold water (3 l) with stirring. Stirring was continued for 3 hours. The aqueous mixture was extracted with diethyl ether. The ether extracts were washed with aqueous sodium hydrogen carbonate solution and then with brine. The extracts were dried over anhydrous magnesium sulphate and then evaporated in vacuo. Distillation gave 2-hydroxymethyl-2-n-propyl propan-1,3-diol triacetate (238 g), a colourless oil (b.pt. 120-140°C, 1.5 mm Hg). Sodium (0.5g) was added to a stirred solution of the above triacetate (238 g) in methanol (2.5 l). The mixture was refluxed, with stirring, for 72 hours. The mixture was evaporated in vacuo. **2-Hydroxymethyl-2-n-propylpropan-1,3-diol** (87 g) was obtained as colourless crystals (m.pt. 93°C). Ref: W.E. Conrad, L.A. Levasseur, R.F. Murphy, N.L. Hare and H.E. Conrad. J. Org. Chem 1962, 27, 2227.

(ii) A mixture of 2-hydroxymethyl-2-n-propylpropan-1,3-diol (24.6 g), diethyl carbonate (20.1 ml), potassium hydroxide (0.3 g) and dry ethanol (2 ml) was heated to gentle reflux (oil bath 110-120°C) under a stream of nitrogen for 30 minutes. After this time the ethanol formed was removed by distillation at atmospheric pressure (oil bath 130-140°C, still head temperature 76°C). The pressure was reduced to 20 mm Hg and the oil bath temperature adjusted to 230°C. **3-Hydroxymethyl-3-n-propyloxetane** distilled as a colourless liquid (16.7 g) (head temperature 120-126°C). Ref: European Patent Application 216624.

(iii) A solution of 3-mercaptopropionic acid (15 ml) in dry dimethylformamide (250 ml) was stirred at 0°C, under nitrogen. Sodium hydride (10.2 g; 80% dispersion in oil) was added carefully and the mixture was stirred at 40°C for 1 hour. The mixture was cooled to 0 ° C and propargyl bromide (60 g ; 80% in toluene) was added dropwise. The mixture was stirred at 20°C for 3 hours and then at 80°C for 1 hour. The mixture was cooled and poured into water. The aqueous mixture was extracted with diethyl ether, and the extracts washed with water and dried over anhydrous magnesium sulphate. The solvent was removed in vacuo. The resulting oil (30 g) (mainly prop-2-ynyl 3-(prop-2-ynylthio) propionate) was added to a solution of sodium hydroxide (8.0 g) in water (100 ml) and methanol (100 ml) and the mixture was stirred at 20°C for 24 hours. The mixture was extracted with diethyl ether and the aqueous solution was acidified with hydrochloric acid. The acidic mixture was extracted with diethyl ether and the ethereal extracts were dried over anhydrous magnesium sulphate. The solvent was removed in vacuo. **3-(Prop-2-ynylthio) propionic acid** was obtained as a red oil (12.0 g) and was used without further purification.

(iv) **4-n-Propyl-1-[2-(prop-2-ynylthio)ethyl]-2,6,7-trioxa bicyclo[2.2.2]octane** was prepared from 3-(prop-2-ynylthio) propionic acid and 3-hydroxymethyl-3-n-propyloxetane using the following methodology:

A mixture of 3-(prop-2-ynylthio) propionic acid and thionyl chloride (1.95 ml) in benzene (25 ml) was heated under reflux for 3 hours. The resulting solution was cooled and then evaporated in vacuo. The acid halide thus obtained was taken up in ether (5 ml) and added, dropwise, to a stirred solution of 3-hydroxymethyl-3-n-propyloxetane (1.2 g) and pyridine (0.8 ml) in dry ether (20 ml). The reaction mixture was stirred at room temperature for 16 hours. After this time the organic phase was washed with water,

5% hydrochloric acid, saturated sodium bicarbonate solution and brine before drying over anhydrous magnesium sulphate and then evaporation in vacuo. The residue was purified by chromatography on silica, pre-eluted with hexane containing 1% triethylamine. Elution with hexane/ether mixtures gave (3-propyloxetan-3-yl) methyl 3-(prop-2-ynylthio) propioniate. Boron trifluoride etherate (0.18 ml) was added to a stirred solution of (3-propyloxetan-3-yl) methyl 3-(prop-2-ynylthio) propioniate in dry dichloromethane (25 ml) at -70°C. The mixture was allowed to warm to room temperature over 16 hours. Triethylamine (0.28 ml) was then added and the solvent was removed in vacuo. The residue was partitioned between diethyl ether and water. The organic phase was separated and further washed with water and brine before drying over anhydrous magnesium sulphate. The solvent was evaporated in vacuo and the residue was purified by column chromatography.

**4-t-Butyl-1-[2-(prop-2-ynylthio) ethyl]-2,6,7-trioxabicyclo [2.2.2]octane** was prepared from 3-(prop-2-ynylthio)propionic acid and 3-t-butyl-3-(hydroxy methyl) oxetane using the same methodology.

**4-n-Propyl-1-[2-(prop-2-ynylthio)ethyl]-2,6,7-trioxabicyclo [2.2.2]octane-3-carbonitrile** was prepared from 3-(prop-2-ynyl thio)-propionic acid and 3-formyl-3-n-propyloxetane using the following methodology:

A solution of dimethyl sulphoxide (12 ml) in dry dichloro methane (4.0 ml) was added to a solution of oxalyl chloride (7.4 ml)in dichloromethane (25 ml) stirred at -70°C under nitrogen. After the addition was complete the resulting mixture was stirred for a further 5 minutes at -70°C before a solution of 3-hydroxymethyl-3-n-propyl oxetane (10.0 g) in dichloromethane (25 ml) was added, dropwise, over 10 minutes. The resulting mixture was allowed to stir for a further 30 minutes when neat triethylamine (54 ml) was added over approximately 30 minutes. The reaction mixture was allowed to warm to room temperature over 3 hours when it was poured into water. The organic phase was separated and the aqueous layer was further extracted with dichloromethane. The combined organic extracts were washed with dilute hydrochloric acid, saturated sodium bicarbonate and brine. The resulting organic phase was dried over anhydrous magnesium sulphate and evaporated in vacuo to give **3-formyl-3-n-propyl oxetane** (10.5 g) (European Patent Application No. 216624) as a yellow oil.

A mixture of 3-(prop-2-ynylthio)-propionic acid and thionyl chloride (1.95 ml) in benzene (25 ml) was heated at reflux for 3 hours. The resulting solution was allowed to cool and then evaporated in vacuo. The acid chloride thus obtained was added to a stirred solution of 3-formyl-3-n-propyloxetane (1.14 g) in ether (50 ml) followed by a solution of sodium cyanide (0.61 g) in water (1 ml). The resulting mixture was stirred briskly at room temperature for 16 hours. After this time the reaction mixture was washed with water, saturated sodium bicarbonate and brine, before drying over anhydrous magnesium sulphate. The solvent was removed in vacuo and the residue was purified by column chromatography. Gradient elution gave **1-cyano-1-(3-propyloxetan-3-yl)-methyl 3-(prop-2-ynylthio)-propionate.**

Boron trifluoride etherate (0.25 ml) was added to a solution of 1-cyano-1-(3-propyloxetan-3-yl)-methyl 3-(prop-2 -ynylthio) propionate in dry dichloromethane (10 ml) stirred at -70°C under a nitrogen atmosphere. The resulting solution was allowed to warm to room temperature overnight. Triethylamine (0.38 ml) was added and the solvent was removed under vacuum. The residue was partitioned between water and diethyl ether. The organic phase was separated and washed with water and brine before drying over anhydrous magnesium sulphate and evaporation in vacuo. The residue was purified by column chromatography on alumina.

**Example II : 4-n-Propyl-1-[2-(prop-2-ynyloxy)ethyl]-2,6,7-trioxabicyclo[2.2.2]octane**

(i) **3-(Prop-2-ynyloxy)propionic acid** was prepared from propargyl alcohol and ethyl 3-bromopropionate using methodology analogous to that describing the synthesis of 3-(but-3-yn-l-yloxy) propionic acid (Example IV).

(ii) **4-n-Propyl-1-[2-(prop-2-ynyloxy)ethyl]-2,6,7-trioxa bicyclo[2.2.2]octane** was prepared from 3-(prop-2-ynyloxy) propionic acid and 3-hydroxymethyl-3-n-propyloxetane using methodology described in Example I.

**4-t-Butyl-1-[2-(prop-2-ynyloxy)ethyl]-2,6,7-trioxabicyclo [2.2.2]octane** was prepared from 3-(prop-2-ynyloxy) propionic acid and 3-t-butyl-3-hydroxymethyloxetane using methodology described in Example I.

**4-n-Propyl-1-[2-(prop-2-ynyloxy)ethyl]-2,6,7-trioxabicyclo [2.2.2]octane-3-carbonitrile** was prepared from 3-(prop-2-ynyloxy) propionic acid and 3-formyl-3-n-propyloxetane using methodology described in Example I for the carbonitrile derivative.

**Example III : 1-(But-3-ynyloxymethyl)-4-n-propyl-2,6,7-trioxa bicyclo[2.2.2]octane**

(i) **2-(But-3-ynyloxy)acetic acid** was prepared from but-3-yn-1-ol and ethyl bromoacetate using methodology analogous to that describing the synthesis of 3-(but-3-ynyloxy)propionic acid (Example IV).

(ii) **1-(But-3-ynyloxymethyl)-4-n-propyl-2,6,7-trioxa bicyclo[2.2.2]octane** was prepared from 2-(but-3-ynyloxy)acetic acid and 3-hydroxymethyl-3-n-propyl-oxetane using methodology described in Example I.

**4-t-Butyl-1-(but-3-ynyloxymethyl)-2,6,7-trioxabicyclo[2.2.2] octane** was prepared from 2-(but-3-ynyloxy)-acetic acid and 3-t-butyl-3-hydroxymethyloxetane using methodology decribed in Example I.

**1-(But-3-ynyloxymethyl)-4-n-propyl-2,6,7-trioxabicyclo[2.2.2] octane-3-carbonitrile** was prepared from 2-(but-3-ynyloxy) acetic acid and 3-formyl-3-n-propyl-oxetane using methodology described in Example I for the carbonitrile derivative.

**1-(But-3-ynyloxymethyl)-4-(prop-2-enyl)-2,6,7-trioxabicyclo [2.2.2]octane-3-carbonitrile** was prepared in an analogous manner, starting from 2-(but-3-ynyloxy)acetic acid (Example III) and 3-formyl-3-(prop-2-enyl)-oxetane.

**Example IV : 1-[2-(But-3-ynyloxy)ethyl]-4n-propyl-2,6,7-trioxabicyclo[2.2.2]octane**

(i) Sodium hydride (4.6 g of 60% dispersion in mineral oil) was added to a stirred solution of but-3-yn-1-ol (16.2 g, 0.23 mol, Lancaster) in dry toluene (200 ml). After stirring for 2 hours at 25°C a solution of ethyl 3-bromopropionate (20.8 g, 0.115 mol, Lancaster) was added. The reaction mixture was stirred and refluxed for 7 hours. **Ethyl 3-(but-3-ynyloxy)propionate** (17.8 g) was obtained by quenching the cooled reaction mixture in water, extracting with ether, washing with brine, drying over anhydrous magnesium sulphate followed by evaporation.

(ii) Ethyl 3-(but-3-ynyloxy)propionate (17.4 g) was stirred overnight with a solution of sodium hydroxide (150ml, 2M). After extracting with ether the aqueous layer was acidified with concentrated hydrochloric acid. The required acid was obtained by ether extraction. The organic phase was washed with brine and dried over magnesium sulphate. Evaporation gave **3-(but-3-ynyloxy) propionic acid** (8.8 g) as a colourless oil.

(iii) **1-[2-(But-3-ynyloxy)ethyl]-4-n-propyl-2,6,7-trioxa bicyclo[2.2.2]octane** was prepared from 3-(but-3-ynyloxy) propionic acid and 3-hydroxymethyl-3-n-propyloxetane using the methodology outlined in Example I.

**1-[1-(But-3-ynyloxy)ethyl]-4-n-propyl-2,6,7-trioxabicyclo [2.2.2]octane** was prepared, using the above methodology and starting from ethyl 2-bromopropionate (supplied by Lancaster Synthesis) and but-3-yn-1-ol (supplied by Lancaster Synthesis),

**Example V: 1-(But-3-ynylthiomethyl)4-n-propyl-2,6,7-trioxa bicyclo[2.2.2]octane**

(i) **But-3-ynyl methanesulphonate**

A solution of methanesulphonyl chloride (23.7 ml) in dry dichloromethane (25 ml) was added dropwise to a solution of but-3-yn-1-ol (Lancaster Synthesis) and triethylamine (47.3 ml) in dichloromethane (300 ml), stirred under a nitrogen atmosphere at -70°C. The resulting mixture was allowed to warm to room temperature over 16 hours. The mixture was then washed with water, dilute hydrochloric acid, saturated sodium bicarbonate solution and brine before drying over anhydrous magnesium sulphate and evaporation in vacuo. But-3-ynyl methanesulphonate was obtained.

(ii) **2-(But-3-ynylthio)acetic acid**

Method A: Thioglycollic acid (6.0 g) was added to a stirred solution of sodium hydroxide (8.0 g) in ethanol (50 ml). But-3-ynyl methanesulphonate (7.4 g) was added and the mixture was stirred at 20°C for 24 hours. The reaction mixture was poured into water and the aqueous mixture was extracted with diethyl ether. The aqueous alkaline extracts were acidified with hydrochloric acid and the mixture was extracted with diethylether. The ethereal extracts were dried over anhydrous magnesium sulphate and evaporated in vacuo. The resulting oil was stirred in dry dimethylformamide (100 ml) and anhydrous sodium carbonate (15 g) was added. Methyl iodide (7.0 ml) was added and the reaction mixture was stirred at 20°C for 3 days. Water was added and the aqueous mixture was extracted with diethyl ether. The ethereal extracts were washed with water and dried over anhydrous magnesium sulphate. The solvent was removed in vacuo and the residue was chromatographed on silica, eluting with 1:4 diethylether:hexane. Methyl 2-(but-3-ynylthio)acetate was obtained as a colourless oil (1.2 g) (a small amount of methyl 2-(but-3-yn-1-ylthio)propionate was present). A mixture of methyl 2-(but-3-ynylthio)-acetate (0.6 g) and sodium hydroxide solution (20 ml, 2N, 1:1 methanol:water) was stirred for 24 hours at

20°C. Water was added and the aqueous mixture was extracted with diethyl ether. The aqueous alkaline extracts were acidified with hydrochloric acid and the mixture was extracted with diethyl ether. The ethereal extracts were dried over anhydrous magnesium sulphate and the solvent was removed in vacuo. 2-(But-3-ynylthio) acetic acid was obtained as a colourless oil (0.5 g). (A small amount of 2-(but-3-ynylthio)propionic acid was present). Method B: 2-(But-3-ynylthio)acetic acid was prepared from thioglycollic acid and but-3-ynyl methanesulphonate using the methodology described in stage (i) of Example I.

(iii) **1-(But-3-ynylthiomethyl)-4-n-propyl-2,6,7-trioxa bicyclo[2.2.2]octane** was prepared from 2-(but-3-ynylthio)acetic acid and 3-hydroxymethyl-3-n-propyl-oxetane using the methodology described in Example I.

**4-t-Butyl-1-(but-3-ynylthiomethyl)-2,6,7-trioxabicyclo[2.2.2] octane** was prepared from 2-(but-3-ynylthio)acetic acid and 3-t-butyl-3-hydroxymethyloxetane using the methodology described in Example I. A small amount of **4-t-butyl-1-[1-(but-3-yn-1-ylthio)ethyl]-2,6,7-trioxabicyclo[2.2.2]octane** was also present.

**Example VI : 1-(But-3-ynyloxymethyl)-4-n-propyl-2,6,7-trithia bicyclo[2.2.2]octane**

i) Methanesulphonyl chloride (74.0 g) was added dropwise over 30 minutes to a solution of 2-hydroxymethyl-2-n-propylpropan-1,3-diol (28.0 g) in dry pyridine (200 ml) under nitrogen at 0°C. The mixture was allowed to warm to room temperature. After stirring for 18 hours the mixture was poured into water (200 ml) and extracted with chloroform (2x200 ml). The chloroform extracts were washed with water (2 x 100 ml), dried over anhydrous magnesium sulphate and evaporated in vacuo to give a brown solid. This was stirred in dry diethyl ether (200 ml) to give **2-hydroxymethyl-2-n-propylpropan-1,3-diol trimethanesulphonate** as a white solid.(70.0 g)(m.pt.103.6°C).

ii) Sodium trithiocarbonate (18.0 g) [see J.Org.Chem. **1968, 33**, 1275] in water (25 ml) was added to a solution of 2-hydroxy methyl-2-n-propyl-propan-1,3-diol trimethanesulphonate (12.0 g) in dimethylformamide (100 ml). The mixture was heated to reflux (130°C) for 4 hours then allowed to cool to room temperature. After a further 18 hours stirring, dilute sulphuric acid solution (50 ml) was added slowly over 30 minutes. The mixture was extracted with chloroform. The extracts were dried over anhydrous magnesium sulphate and evaporated in vacuo to give a brown liquid. Hexane (200 ml) was added and the mixture washed with water (3 x 50 ml). Drying over anhydrous magnesium sulphate and evaporation gave an amber oil (6.8 g). The crude oil (6.4 g) in diethyl ether (10 ml) was added dropwise to a suspension of lithium aluminium hydride (3.0 g) in dry diethyl ether (100 ml) at a rate sufficient to maintain reflux. The mixture was stirred for a further hour after addition was complete, then water (3 ml) was added carefully. Dilute sulphuric acid (3 ml) was added and was followed by water (3 ml). The mixture was filtered, the solid washed with diethyl ether (50 ml) and the combined filtrates dried over anhydrous magnesium sulphate and evaporated in vacuo to give **2-mercaptomethyl-2-n-propyl-propan-1,3-dithiol** as a pale yellow oil (5.3 g).

iii) Hydrogen chloride gas was bubbled through a suspension of paraformaldehyde (4.3 g) in but-3-yn-1-ol (10.0 g) (Aldrich) and dry dichloromethane (30 ml) at -20°C for 30 minutes. The mixture was allowed to warm to room temperature then stirred for a further 18 hours. Cold water (30 ml) was added and the organic layer separated, dried over anhydrous calcium chloride and evaporated to give **but-3-ynyl chloromethyl ether** as an amber liquid (14.2 g).

iv) 2-Mercaptomethyl-2-n-propylpropan-1,3-dithiol (2.3g) and triethylorthoformate (1.74 g) were heated together in dry toluene (5 ml) containing p-toluenesulphonic acid (5 mg) and the ethanol produced distilled off. After cooling, toluene (15 ml) was added and the mixture washed with water (2 x 10 ml). Drying over anhydrous magnesium sulphate and evaporation in vacuo gave a pale yellow oil. This was extracted with diethyl ether (50 ml) and the extracts evaporated in vacuo to give a white solid. Purification by chromatography on alumina, eluting with 1:10 dichloromethane:hexane saturated with ammonia gave **4-n-propyl-2,6,7-trithiabicyclo[2.2.2]octane** as a white solid (0.33 g, m.pt.139 °C). Mass spectrum (Chemical Ionisation) M + 1 207.

v) n-Butyllithium (0.3 ml,1.6M solution in hexane) was added to a solution of 4-n-propyl-2,6,7-trithiabicyclo [2.2.2]octane (0.1 g) in dry tetrahydrofuran (5 ml) at -70°C, under nitrogen. The solution was stirred for 30 minutes and but-3-ynyl chloromethyl ether (0.058 g) in dry tetrahydrofuran (2.0 ml) was added and the reaction mixture was allowed to warm to 20°C. Water (10 ml) was added and the mixture was extracted with diethyl ether. The ethereal extracts were dried over anhydrous magnesium sulphate and the solvent was removed in vacuo. The residue was purified by chromatography on alumina, eluting with 1:10 dichloromethane:hexane saturated with ammonia. **1-(But-3-ynyloxymethyl)-4-n-propyl-2,6,7-**

**trithiabicyclo[2.2.2]octane** was obtained as a waxy solid (0.032 g).

**Formulations**

**1. Emulsifiable Concentrate**

| | |
|---|---|
| Compound of formula (I) | 10.00 |
| Ethylan KEO | 20.00 |
| Xylene | 67.50 |
| Butylated Hydroxyanisole | 2.50 |
| | 100.00 |

**2. Wettable Powder**

| | |
|---|---|
| Compound of formula (I) | 25.00 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

**3. Dust**

| | |
|---|---|
| Compound of formula (I) | 0.50 |
| Butylated Hydroxyanisole | 0.10 |
| Talc | 99.40 |
| | 100.00 |

**4. Bait**

| | |
|---|---|
| Compound of formula (I) | 40.25 |
| Icing Sugar | 59.65 |
| Butylated hydroxy toluene | 0.10 |
| | 100.00 |

**5. Lacquer**

| | |
|---|---|
| Compound of formula (I) | 0.1 |
| Piperonyl Butoxide | 0.5 |
| Butylated Hydroxyanisole | 10.1 |
| High aromatic white spirit | 92.0 |
| | 100.00 |

**6. Aerosol**

| | |
|---|---|
| Compound of formula (I) | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

**7. Spray**

| | |
|---|---|
| Compound of formula (I) | 0.1 |
| Butylated Hydroxyanisole | 0.1 |
| Xylene | 10.0 |
| Odourless Kerosene | 89.8 |
| | 100.00 |

**8. Potentiated Spray**

| | |
|---|---|
| Compound of formula (I) | 0.1 |
| Piperonyl Butoxide | 0.5 |
| Butylated Hydroxyanisole | 0.1 |
| Xylene | 10.1 |
| Odourless Kerosene | 89.2 |
| | 100.00 |

**Biological activities**

The following examples illustrate, in a non-limiting manner, the pesticidal activity of compounds of formula (I).

**Spray Tests**

The activity of the compounds of the invention were tested by dissolving the compounds in acetone (5%) and then diluting in water: "Synperonic" (94.5%: 0.5%) to give a water emulsion. The solution was then used to treat the following insects.

Musca domestica

20 female Musca were contained in a cardboard cylinder with gauze over both ends. Solution containing the compound was sprayed onto the insects so enclosed and mortality assessed after 48 hours at 25°C.

The following compounds were active at <1000 ppm: 3, 4, 5, 6, 7, 8, 9, 10, 13, 14.

The following compound was active at <200 ppm: 15.

Sitophilus granarius

20 adult Sitophilus were added to 10g wheat which had been previously treated with 2ml of the solution containing the compounds. Mortality was assessed after 6 days at 25°C.

The following compounds were active against Sitophilus granarius at <1000 ppm: 2, 5, 6, 8, 9, 10, 13, 14.

The following compound was active against Sitophilus granarius at <200 ppm: 15.

Myzus persicae

10 adult Myzus were placed on a leaf disc of chinese cabbage. 24 hours later the disc was sprayed with a solution containing the compound. Mortality was assessed after 2 days at 25°C.

The following compound was active at <1000 ppm: 12.

The following compound was active at <200 ppm: 15.

Plutella xylostella

7 Plutella larvae were sprayed with the solution containing the compound and added to a chinese cabbage leaf which had been similarly sprayed and left to dry. Alternatively, 8-10 Plutella larvae were put onto leaf discs and sprayed with the solution containing the compound. Mortality was assessed after 2 days at 25°C.

The following compounds were active at <1000 ppm: 8, 11.

**Additional spray tests**

The activities of the compounds were investigated further. The compounds were dissolved in acetone (75%) and water (25%) was added. The solution was then used to spray the following insects:

Aphis fabae

A mixed population of Aphis fabae was tested on Nasturtium leaf.

The following compounds were active at <1000 ppm: 8,9.

Macrosteles fascifrons

Adult Macrosteles fascifrons were tested on wheat seedlings.

The following compounds were active at <1000 ppm: 6, 8, 9.

Diabrotica undecimpunctata

3rd Instar Diabrotica undecimpunctata were tested on filter paper.

The following compounds were active at <1000 ppm: 6, 8, 9.

**Topical Application Tests**

The activity of compounds of the invention against anaesthetised male Periplaneta americana was demonstrated by the topical application to the test insect of a solution of the compound under test in butanone. Mortality was assessed after 6 days.

The following compound was active at <50 µg: 8.

The activity of compounds of the invention against anaesthetised male Blattella germanica was demonstrated by topical application to the test insect of a solution of the compound under test in butanone. Mortality was assessed after 6 days.

The following compounds were active at <5 µg: 3, 15.

Table I

| Trioxabicyclooctanes (Y = Y¹ = Z = O) | | | | |
|---|---|---|---|---|
| Compound | A-X | $R^1$ | $R^2$ | Example |
| 01 | 2-(prop-2-ynylthio)ethyl | n-Pr | H | I |
| 02 | 2-(prop-2-ynylthio)ethyl | t-Bu | H | I |
| 03 | 2-(prop-2-ynylthio)ethyl | n-Pr | CN | I |
| 04 | 2-(prop-2-ynyloxy)ethyl | n-Pr | H | II |
| 05 | 2-(prop-2-ynyloxy)ethyl | t-Bu | H | II |
| 06 | 2-(prop-2-ynyloxy)ethyl | n-Pr | CN | II |
| 07 | But-3-ynyloxymethyl | n-Pr | H | III |
| 08 | But-3-ynyloxymethyl | t-Bu | H | III |
| 09 | But-3-ynyloxymethyl | n-Pr | CN | III |
| 10 | But-3-ynyloxymethyl | 2-propenyl | CN | III |
| 11 | 2-(But-3-ynyloxy)ethyl | n-Pr | H | IV |
| 12 | 1-(But-3-ynyloxy)ethyl | n-Pr | H | IV |
| 13 | But-3-ynylthiomethyl | n-Pr | H | V |
| 14 | But-3-ynylthiomethyl | t-Bu | H | V |
| | | | | |

Table II

| Trithiabicyclooctanes (Y = Y¹ = Z = S) | | | | |
|---|---|---|---|---|
| Compound | A-X | $R^1$ | $R^2$ | Example |
| 15 | But-3-ynyloxymethyl | n-Pr | H | VI |

**Table III - Characterising Data for Trioxabicyclooctanes**

| Compound No. | m.p. | | NMR Spectrum (TMS/$CDCL_3$ ppm) (Multiplicity JHz) |
|---|---|---|---|
| 01 | Oil | 257 | 3.95,6H,s;3.25,2H,d,1.5;2.85,2H,m; 2.20,1H,t,1.5;2.05,2H,m;1.20,4H,m; 0.90,3H,t,7. |
| 02 | 76-82° | 271 | 4.00,6H,s;3.25,2H,d,1.5;2.80,2H,m; 2.20,1H,t,1.5;2.00,2H,m;0.90,9H,s. |
| 03 | Oil | 282 | 4.80,1H,d;4.20,1H,m;4.00,3H,m;3.25, 2H,d,1.5;2.80,2H,m;2.25,1H,t,1.5; 2.05,2H,m;1.30,4H,m;0.95,3H,t,7 |
| 04 | Solid <35° | 241 | 4.15,2H,d;3.90,6H,s;3.65,2H,t,6; 2.40,1H,t;2.00,2H,t,6; 1.20,4H,m; 0.90,3H,t,6 |
| 05 | Oil | 255 | 4.15,2H,d;4.00,6H,s;3.65,2H,t;2.4, 1H,t;2.05,2H,t;1.90,9H,s |
| 06 | Oil | 266 | 4.80,1H,d,2.7; 4.2-3.9,6H,m; 3.65,2H,t,5; 2.45,1H,t; 2.2,2H,t,5; 1.4-1.15,4H,m; 0.9,3H,t. |
| 07 | 64°C | 241 | 4.00,6H,s;3.70,2H,t,6;3.55,2H,s; 2.55,2H,td;1.95,1H,t;1.25,4H,m; 0.90,3H,t,7. |

**Table III - Characterising Data for Trioxabicyclooctanes**

| Compound No. | m.p. | | NMR Spectrum ($TMS/CDCL_3$ ppm) (Multiplicity JHz) |
|---|---|---|---|
| 08 | Oil | 255 | 4.05,6H,s;3.7,2H,t;3.55,2H,s;2.5, 2H,td,1.95,1H,t;0.85,9H,s. |
| 09 | 67.4° | 266 | 4.8,1H,d,2.65;4.25,1H,m;4.10-3.95, 3H,m;3.70,2H,t;3.60,2H,s;2.50,2H,td; 2.00,1H,t;1.45-1.20,4H,m;1.0,3H,t,7. |
| 10 | Oil | 264 | 5.55,1H,m;5.22,2H,m;4.82,1H,d;4.25, 1H,dd;4.00,3H,m;3.75,2H,t;3.60,2H,s; 2.50,2H,td;2.20,2H,d;1.97,1H,t. |
| 11 | Oil | 255 | 3.9,6H,s;3.60,2H,m;3.55,2H,t,7;2.45, 2H,td,7 and 2.6;2.00,2H,m;1.95,1H,t, 2.6;1.15,4H,m;0.90,3H,t,6.8. |
| 12 | Oil | 255 | 3.95,6H,s;3.83,1H,m;3.65,1H,m;3.46, 1H,m;2.45,2H,m;3.46,1.95,1H,t,2.6; 1.20-1.15,7H,m;0.90,3H,t. |
| 13 | 62° | 257 | 3.95,6H,s;2.85,2H,t,7;2.80,2H,s;2.5, 2H,td;2.00,1H,t;1.40,4H,m;0.90,3H,t. |
| 14 | Oil | 271 | 4.05,6H,s;2.83,2H,t,7;2.80,2H,s;2.5, 2H,td;2.00,1H,t;0.85,9H,s. |

Table IV

| Characterising Data for Trithiabicyclooctanes | | | |
|---|---|---|---|
| Compound No. | m.p. | | NMR Spectrum ($TMS/CDCL_3$ ppm) (Multiplicity JHz) |
| 15 | Oil | 289 | 3.85,2H,s;3.75,2H,t;3.00,6H,s;2.50, 2H,m;1.97,1H,t;1.40,4H,m;0.97,3H,t. |

## Claims

1. A compound of the formula (I):

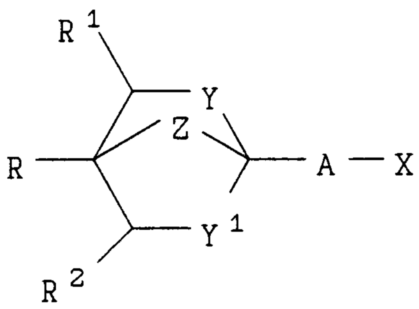

(I)

wherein R is a $C_{2-10}$ non-aromatic hydrocarbyl group optionally substituted by, or methyl substituted by cyano, halo, $C_{1-4}$ alkoxy optionally substituted by halo, or a group $S(O)_mR^3$ where $R^3$ is $C_{1-4}$ alkyl optionally substituted by halo and m is 0, 1 or 2, or R is phenyl optionally substituted by $C_{1-4}$ alkoxy, $C_{1-3}$ alkyl, $C_{2-4}$ alkynyl, halo, $C_{1-4}$ haloalkyl, cyano or a group $S(O)_mR^3$ as defined hereinbefore;

$R^1$ and $R^2$ may be the same or different, and each is hydrogen, halo, or a $C_{1-3}$ aliphatic group optionally substituted by halo, cyano, $C_{1-5}$ carbalkoxy, $C_{1-4}$ alkoxy, or a group $S(O)_{m'}R^4$ wherein m' is O, 1 or 2 and $R^4$ is $C_{1-4}$ alkyl; cyano, gem-dimethyl, or $C_{1-5}$ carbalkoxy, or $R^1$ and R and the carbon atoms to which they are attached form a $C_{5-7}$ carbocyclic ring optionally substituted by halo, or a $C_{1-3}$ aliphatic or alkoxy group;

A is a $C_{5-8}$ non-aromatic hydrocarbyl group which contains one hetero atom selected from oxygen or sulphur and terminates in a C≡C fragment adjacent to X;

X is hydrogen;

Y and Y1 are the same or different and are each selected from oxygen and $S(O)_{n'}$ where n' is 0, 1 or 2; and Z is $CH_2O$ or $CH_2S(O)_{n''}$ wherein n'' is 0, 1 or 2.

2. A compound of the formula (I) according to claim 1 wherein R is n-propyl, n-butyl i-butyl, t-butyl or phenyl.

3. A compound of the formula (I) according to claim 1 or 2 wherein R1 and R2 are independently selected from hydrogen, methyl, cyano or trifluoromethyl.

4. A compound of the formula (I) according to any of claims 1 to 3 wherein A is a $CH_2O(CH_2)_2C{\equiv}C$ group.

5. A compound selected from:
4-t-Butyl-1-[2-(prop-2-ynylthio)ethyl]-2,6,7-trioxabicyclo [2.2.2]octane,
4-n-Propyl-1-[2-(prop-2-ynylthio)ethyl]-2,6,7-trioxabicyclo [2.2.2]octane,
4-t-Butyl-1-[2-(prop-2-ynyloxy)ethyl]-2,6,7-trioxabicyclo [2.2.2]octane,
4-n-Propyl-1-[2-(prop-2-ynyloxy)ethyl]-2,6,7-trioxabicyclo [2.2.2]octane-3-carbonitrile,
1-(But-3-ynyloxymethyl)-4-n-propyl-2,6,7-trioxabicyclo [2.2.2]octane,
4-t-Butyl-1-(but-3-ynyloxymethyl)-2,6,7-trioxabicyclo [2.2.2]octane,
1-(But-3-ynyloxymethyl)-4-(prop-2-enyl)-2,6,7-trioxabicyclo [2.2.2]octane-3-carbonitrile,
1-[2-(But-3-ynyloxy)ethyl]-4-n-propyl-2,6,7-trioxabicyclo [2.2.2]octane,
1-(But-3-ynylthiomethyl)-4-n-propyl-2,6,7-trioxabicyclo [2.2.2]octane,
4-t-Butyl-1-(but-3-ynylthiomethyl)-2,6,7-trioxabicyclo [2.2.2]octane,
1-(But-3-ynyloxymethyl)-4-n-propyl-2,6,7-trithiabicyclo [2.2.2]octane.

6. A pesticidal formulation comprising a compound of the formula (I) as defined according to any of the preceding claims in admixture with one or more carriers or diluents.

7. A pesticidal formulation according to claim 6 which additionally contains a synergist or potentiator.

8. A pesticidal formulation according to either claim 6 or claim 7 which additionally contains one or more pesticidally active ingredients, attractants, repellents, bacteriocides, fungicides and/or anthelmintics.

9. A compound of the formula (I) for use in human or veterinary medicine.

10. A method for the control of arthropod or helminth pests which comprises administering to the arthropod or helminth or their environment an effective amount of a compound of the formula (I) as prepared according to any one of the preceding claims.

11. A method for the control of pesticidal infestations on plants and/or stored products and/or an environment which comprises administering an effective amount of a compound of the formula (I) as defined according to any one of the preceding claims to the plant and/or stored product and/or an environment susceptible to pest infestation.

12. A process for the manufacture of a compound of the formula (I) as defined according to claim 1 which comprises:
i) for the production of compounds wherein Y and $Y^1$ are oxygen and Z is $CH_2O$:
a) the cyclisation of a compound of the formula (II):

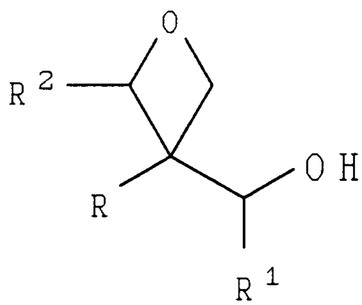

(III)

wherein R to $R_2$, A and X are as defined according to claim 1, in the presence of an acid catalyst or;
b) the reaction of a strong base with a compound of the formula (VII):

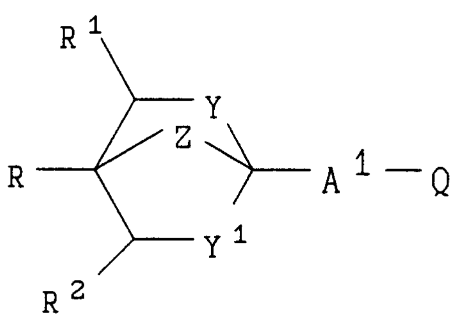

(VII)

wherein R to $R^2$ and A are as defined according to claim 1, $A^1$ is defined such that $A^1$-C≡C forms the group A and Q is a group capable of conversion into an ethynyl group or,
ii) for the production of compounds wherein n' is 0, $Y^1$ is O or S, Y is O or S, Z is $CH_2S$ or $CH_2O$, the reaction of a compound $(AlkO)_3C{\equiv}CAX$ with a compound of the formula (XIV):

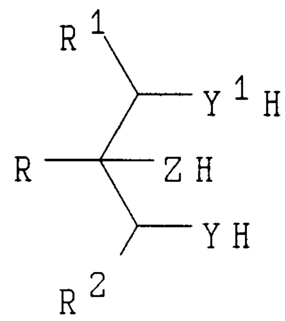

(XIV)

wherein R to $R^2$, A, X, Y, $Y^1$ and Z are as defined according to claim 1 and Alk is a $C_{1-4}$ alkyl group or;

iii) for the production of compounds wherein $Z=CH_2S$ or $CH_2O$ and Y and $Y^1$ are sulphur the reaction of a compound $L^2AX$ with a compound of the formula (XV):

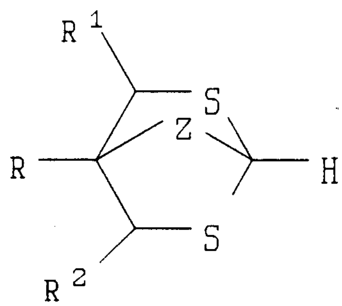

(XV)

wherein R to $R^2$, A and X are as defined according to claim 1, Z is $CH_2S$ or $CH_2O$ and $L^2$ is a leaving group, or;

iv) the conversion of a compound of the formula (I) into another compound of the formula (I).

**13.** A compound of formulas (II) (VII), (XIV) or (XV).